# EUROPEAN PATENT SPECIFICATION

(11) **EP 3 615 127 B1**
(45) Date of publication and mention of the grant of the patent: **26.08.2026**
(21) Application number: 18828803.9
(22) Date of filing: 03.07.2018
(51) Int. Cl.: A61M 25/10, A61B 17/02, A61B 17/12, A61B 17/43, A61D 19/04

(54) **APPARATUS FOR EVERTING CATHETER WITH ALIGNMENT AND COMPLIANT PRESSURIZATION**
VORRICHTUNG FÜR UMWENDEKATHETER MIT AUSRICHTUNG UND KONFORMEM DRUCKAUFBAU
APPAREIL POUR RETOURNER UN CATHÉTER AVEC ALIGNEMENT ET PRESSURISATION FLEXIBLE

(30) Priority: 03.07.2017 US 201762528422 P
(43) Date of publication of application: 04.03.2020
(73) Proprietor: CrossBay Medical, Inc., San Diego CA 92128 (US)
(72) Inventor: BACICH, Steven R., Half Moon Bay California 94019 (US); VIDYARTHI, Piush, San Rafael California 94901 (US); YUREK, Matthew Thomas, San Diego California 92129 (US); GREELIS, Jack, Carlsbad California 92009 (US); FONTANA, Cristiano Danilo Maria, 20133 Milan (IT)
(74) Representative: Lorenz Seidler Gossel Part. mbB
(86) International application number: PCT/US2018/040820
(87) International publication number: WO 2019/010244

(56) References cited:
- EP-A2- 0 359 489
- US-A- 4 323 072
- US-A- 5 163 927
- US-A- 5 259 836
- US-A- 5 364 345
- US-A- 5 383 889
- US-A- 5 630 797
- US-A- 6 039 721
- US-A1- 2003 023 263
- US-A1- 2013 060 234
- US-A1- 2013 060 234
- US-A1- 2015 066 068
- US-A1- 2015 133 727
- US-A1- 2015 133 727
- US-A1- 2015 142 045
- US-B1- 6 379 372
- US-B1- 6 379 372
- US-B1- 6 530 898

## Description

### BACKGROUND

This application has particular utility for everting catheters that are characterized with an inner catheter, outer catheter, and everting membrane that is connected to both catheters. The inner catheter may contain an inner lumen to pass fluid or media, drugs or therapeutic agents, instruments or devices, and other catheters.

For example, US 2015/133727 A1 discloses a catheter system having a first catheter and a second catheter slidably located in the first catheter, and an everting balloon attached to the first catheter and having a length past the distal end of the first catheter when said first catheter is in the extended configuration. Similar catheter systems are known from document US 2015/142045 A1, US 5364345 A, US 5383889 A, US 6379372 B1, US 2013/060234 A1 and US 6530898 B1.

For physicians and medical professionals, accessing systems for vessels and bodily cavities in patients have typically used various guidewire and catheter technologies or everting catheters. Everting catheters utilize a traversing action in which a balloon is inverted and with the influence of hydraulic pressure created by a compressible or incompressible fluid or media, rolls inside out or everts with a propulsion force through the vessel. Everting balloons have been referred to as rolling or outrolling balloons, evaginating membranes, toposcopic catheters, or linear everting catheters such as those in U.S. Patent Nos. 5,364,345; 5,372,247; 5,458,573; 5,472,419; 5,630,797; 5,902,286; 5,993,427; 6,039,721; 3,421,509; and 3,911,927. These are categorized as everting balloons and are for traversing vessels, cavities, tubes, or ducts in a frictionless manner. In other words, an everting balloon can traverse a tube without imparting any shear forces on the wall being traversed. Because of this action and lack of shear forces, resultant trauma can be reduced and the risk of perforation reduced. In addition as a result of the mechanism of travel through a vessel, material and substances in the proximal portion of the tube or vessel are not pushed or advanced forward to a more distal portion of the tube or vessel.

In addition, as the everting catheter deploys inside out, uncontaminated or untouched balloon material is placed inside the vessel wall. In the inverted or undeployed state, the balloon is housed inside the catheter body and cannot come into contact with the patient or physician. As the balloon is pressurized and everted, the balloon material rolls inside out without contacting any element outside of the vessel. Another advantage of an everting balloon catheter is that the method of access is more comfortable for the patient since the hydraulic forces "pull" the balloon membrane through the vessel or duct as opposed to a standard catheter that needs to be "pushed" into and through the vessel or duct.

Everting catheters have been described as dilatation catheters. Representative examples of dilating everting catheters include U.S. Patent Nos. 5,364,345 and 4,863,440.

Everting catheters have also been described with additional elements such as a handle for controlling instruments within an everting catheter. A representative example is U.S. Patent No. 5,346,498. Everting balloon catheters can be constructed with an inner catheter with an internal lumen or through-lumen (or thru-lumen). The through-lumen can be used for the passage of instruments, media, materials, therapeutic agents, endoscope, guidewires, or other instruments. Representative samples of everting catheters with through-lumens are in US Patent No. 5,374,247 and 5,458,573. In addition, everting catheters have been described with waists or a narrowing of the balloon diameter, such as in U.S. Patent No. 5,074,845.

Furthermore, infertility is a condition that affects 1 out of 8 couples in the US. One of the early treatments in the infertility regime is insemination. Intrauterine insemination or IUI is a very common procedure since it is in the early work up of an infertile couple. Most assisted reproductive clinics perform at least 3 IUI cycles before trying more expensive treatment options such as IVF.

Also, when delivering the reproductive material, such as an embryo, into the uterine cavity, vacuum effect can unintentionally remove the reproductive material from the uterine cavity. In existing systems, when the transfer catheter is retracted from a second outer or guiding catheter (e.g., the "inner" catheter), the retraction produces vacuum pressure within the uterine cavity. This vacuum pressure is created in the uterine cavity by the removal and backward movement of the transfer catheter within the inner catheter. After the embryo transfer is completed, an embryologist may inspect the transfer catheter to verify that the embryos or reproductive material was indeed deposited in the uterus and not pulled back into the transfer catheter because of the vacuum effect. The same procedure may be done for the outer catheter once this catheter is removed.

The passage of the embryo transfer catheter may become impeded if the everting membrane is rotated or twisted. Twists within the balloon membrane can also reduce the ability of the everting membrane to traverse a lumen or cavity or unroll as intended. A twist in the balloon membrane can occur if the inner catheter is rotated about its central axis in relation to a stationary outer catheter. By rotating the inner catheter, the balloon membrane which is connected between both the outer catheter and inner catheter becomes twisted. In this particular situation of an everting balloon, twists in the balloon membrane can significantly impact performance of the everting system.

A twist in the everting membrane can occur during use or prep of the catheter prior to inserting the device within a patient. A twist in the everting membrane can also occur when a catheter system has the requirement of multiple eversions and retractions to complete a procedure within a patient. Likewise, a twist in the balloon system can unintentionally occur as a byproduct of the manufacturing process.

In the device configuration using a handle system, an anti-rotation feature can be particularly advantageous. As described previously, handles are very useful for driving the inner catheter and controlling the advancement and retraction of instruments, other catheters, media, and materials within the inner catheter lumen. Manipulation of a handle can inadvertently rotate the inner catheter system within the outer catheter and thereby creates twists in the balloon membrane. This situation can be exasperated by the introduction and removal of multiple instruments and devices within the inner catheter lumen.

Having an everting catheter system in which twists or inadvertent rotations of the balloon membrane will enable more stable and secure use of an everting catheter. An untwisted balloon membrane provides the least obstructed passage within the everting system. Some everting catheter systems will be more prone to balloon twisting due to the length of the balloon membrane and inner catheter and type of balloon membrane material. In some clinical applications, more tortuous anatomy may instigate a greater likelihood of balloon twists as a result of the manipulations the clinician may need to perform to complete the procedure or obtain access to the desired target location in the body.

Maintaining the alignment of the inner catheter, outer catheter, and balloon membrane may be accomplished through a handle and ratchet system as described previously. The alignment feature is accomplished by the ratchet and handle that prevents rotation of the inner catheter. The systems described herein are directed towards internal catheter apparatus that provide alignment or anti-rotation capability without requiring an additional set of components like rails, tracks, ratchets, or handles on the exterior for the catheter system.

Another clinical issue with an everting catheter is that physicians may inadvertently pull or elongate the inner catheter upon inversion of the balloon membrane. Over-elongation can stretch the balloon membrane or damage the catheter components. A feature that mechanically prevents this from occurring will be a benefit to the catheter system.

Another problematic issue for everting catheters is the pressurization step in prepping the catheter. One option that is described in the prior art is the use of an inflation device with pressure gauge that indicates the internal pressure of the catheter system. Inflation devices with pressure gauges, or building an integral pressure gauge within the catheter system, can be expensive. Using a separate, reusable pressure gauge adds to the number of components required for performing the procedure. Having a simple mechanism that regulates and indicates the amount of pressure within the catheter system would be a benefit. For more specialized procedures, being able to modulate the internal pressure depending upon the medical procedure could be particularly advantageous.

For everting catheters used in IVF procedures, it is beneficial to stabilize the inner catheter when full eversion is completed for two-stage embryo transfer procedures. A two-stage embryo transfer is performed by everting the membrane across the endocervical canal and into the uterine cavity and subsequently placing the loaded embryo transfer catheter through the inner catheter and ultimately within the uterus. This operation is done in two steps and the infertility specialist will inform the embryologist that the inner catheter has been everted and is now in place within the uterine cavity. The embryologist will then aspirate and load the embryo or embryos into the distal end of the embryo transfer catheter for eventual insertion through the inner catheter for deposition in to the uterine cavity. This is the completion of the second stage of the process. During the loading step performed by the embryologist, a mechanism that stabilizes and indicates to the user that the inner catheter is in position would be a benefit.

Another problem with everting catheters is preparing the system by internal pressurization. This preparation step can vary among users and over-pressurization, and under-pressurization, of the everting system can negatively impact the performance of the device.

Another improvement to embryo transfer procedures would be systems that facilitate the use of transvaginal ultrasound. Systems that also remove the requirement for a speculum would be a benefit for patient comfort.

Another area of improvement is accessories that make handling the embryo transfer catheter easier for the embryologist and physician performing the transfer procedure.

### SUMMARY

To address the aforementioned problems, the present invention provides for a system for delivering matter into the reproductive tract of a female as defined by claim 1. Preferred embodiments of the invention are defined by the dependent claims. More particularly, an everting balloon system is disclosed that can be used for uterine access procedures. The everting balloon system can be used for IVF and intrauterine insemination procedures, urinary incontinence diagnostic and therapeutic procedures, delivering intra-fallopian tube inserts, media, or diagnostic instruments, dilation of a body lumen, for access and sealing within a body cavity, or combinations thereof. The system can have a handle for insertion.

The everting balloon system can be used to access the uterus, bladder, ureters, kidneys, ducts, vessels of the vasculature, nasal passageways, other bodily lumens, or combinations thereof. Devices, tools, instrumentation, endoscopes, drugs, therapeutic agents, sampling devices (brushes, biopsy, and aspiration mechanisms), or combinations thereof can be delivered through the inner catheter lumen to the target site.

The everting balloon system has an internal alignment mechanism that prevents rotation and spinning of the balloon membrane.

The everting balloon system can have an internal mechanism that prevents over-elongation of the inner catheter during balloon inversion.

The everting balloon system can have a compliant pressurization apparatus that's provides a pre-determined pressure within the catheter system with an indicator to the user that system is at the appropriate operating pressure.

Another embodiment can automatically pressurize the everting balloon system to a predetermined amount.

The everting balloon system can have an integral pressurization system that provides an indicator and the ability to quickly shift the pressurization state of the balloon system from pressurized to non-pressurized. Intermediate degrees of pressurization can also be selected.

The everting balloon system can have a mechanism that stabilizes the inner catheter at the full eversion stage and provides an indicator to the user that catheter system is at the appropriate step in the process for embryo transfer.

The everting balloon system can have a proximal hub connector that aids the physician and embryologist in delivering the embryo transfer catheter to the delivery catheter.

The everting balloon system can be shaped with distal end features that facilitate uterine access without the need for a speculum and/or tenaculum.

The everting catheter system can have accessories that make the handling of the embryo transfer catheter easier.

### BRIEF DESCRIPTION OF THE DRAWINGS

Figures 1A through 1E are longitudinal cross-sectional views of the distal end of a variation of a method for using the everting balloon system.
Figure 2A illustrates an everting balloon system with a delivery catheter, embryo transfer catheter, and a pressurization syringe in a disassembled configuration.
Figure 2B illustrates a variation of the everting balloon system in an assembled and fully everted configuration.
Figure 2C illustrates the everting balloon system of Figure 2B with the embryo transfer catheter beyond the distal end of the everting balloon membrane.
Figure 3A illustrates cross-sectional view of a variation of a method for using the everting balloon system with a flexible tip guidance wire beyond the distal end of the everting balloon membrane during the eversion process directing the everting balloon system beyond a cul-de-sac in the endocervical canal.
Figure 3B illustrates a cross-sectional view of the method shown in Figure 3A with the flexible tip guidance wire beyond the distal end of the everting balloon membrane at the completion of the eversion process beyond a cul-de-sac in the endocervical canal.
Figure 4 illustrates a variation of the everting balloon system with a stopcock configuration for maintaining pressurization.
Figure 5A illustrates is a close-up view of a variation of the everting balloon system with an internal alignment mechanism that prevents rotation and spinning of the balloon membrane.
Figure 5B is a cross-sectional axial view of a variation of the internal alignment mechanism and mating geometry of the delivery catheter tubing.
Figure 5C illustrates a variation of the alignment piece.
Figure 6 illustrates a variation of an everting balloon system with an internal mechanism that prevents over-elongation of the inner catheter during balloon inversion.
Figures 7A, 7B and 7C illustrate everting balloon systems with a compliant pressurization apparatus which provides a pre-determined pressure within the catheter system with an indicator to the user that system is at the appropriate operating pressure.
Figure 8 illustrates a variation of the everting balloon system with a mechanism that automatically pressurizes the everting balloon system to a predetermined amount.
Figure 9A illustrates a variation of the everting balloon system with an integral pressurization system that provides an indicator and the ability to quickly shift the pressurization state of the balloon system from pressurized to non-pressurized at the fully everted state of the everting balloon system.
Figure 9B illustrates a variation of the everting balloon system with an integral pressurization system that provides an indicator and the ability to quickly shift the pressurization state of the balloon system from high pressurization to low pressurization, and back to high pressurization, or multiple intermediate states of pressurization, during the eversion process.
Figure 10 illustrates a variation of the everting balloon system with a mechanism that stabilizes the inner catheter at the full eversion stage and provides an indicator to the user that catheter system is at the appropriate step in the process for embryo transfer.
Figure 11 illustrates an everting balloon system with a proximal hub connector that aids the physician and embryologist in delivering the embryo transfer catheter to the delivery catheter.
Figures 12A and 12A' illustrates a variation of an everting balloon system shaped with distal end features that facilitate uterine access without the need for a speculum.
Figure 12B illustrates in an axial view of the distal end (e.g., an acorn tip) features that facilitate uterine access without the need for a speculum.
Figure 13 illustrates in a side view of an everting balloon system with a handle that controls the translation of the inner catheter.
Figures 14A and 14A' illustrate a variation of the everting catheter balloon system with a translatable and adjustable distal end tip that can alter the working length of the everting balloon.
Figures 14B and 14B' illustrate the translatable and adjustable distal end tip at an extended position with resultant working length of the everting balloon.
Figures 15A and 15A' illustrate a protective tube system for the embryo transfer catheter that facilitates handling and transport of the catheter.
Figure 15B, 15B', and 15B" illustrate a protective tube system for the embryo transfer catheter in the detached configuration for the loading of embryos.
Figure 15C illustrates a protective tube system for the embryo transfer catheter in the re-attached mode for the transport of the embryo transfer catheter.

### DETAILED DESCRIPTION

An everting balloon system 2 (also referred to as an everting catheter system) that can be used to traverse a vessel, such as the cervical canal is disclosed. The everting balloon system 2 can be used to access the uterine cavity via the cervix. The cervical canal is a single lumen vessel that can stretch or dilate. The everting balloon system 2 can have a control system that can be operated with one hand. The pressurization states of the everting catheter system 104 can be changed and controlled with one hand of the user.

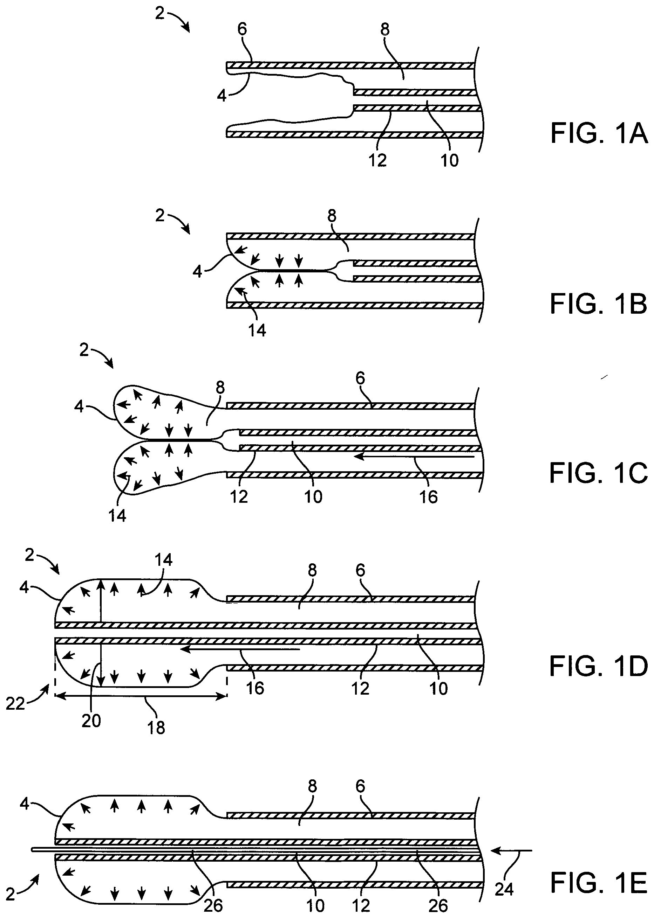

Figures 1A through 1E illustrate that an everting catheter system 104 can have a radially outer catheter 6, a balloon membrane, and a radially inner catheter 12. The inner catheter 12 can have an inner catheter lumen 10 (e.g., a through-lumen). The distal end of the inner catheter lumen 10 can be open or closed. The inner catheter 12 can have the inner catheter lumen 10, or be a solid rod or flexible mandrel, or contain multiple lumens for the delivery of other agents, tools, catheters, instruments, endoscopes, and other media. The inner catheter 12 can be made from multiple polymeric materials and have a more flexible distal end and more rigid proximal end. Distal end flexibility can be enhanced with the incorporation of a distal end coil or spring to provide distal end flexibility and support from kinking the lumen of the inner catheter 12. The internal lumen of the inner catheter 12 can be made from a lubricious material such as Teflon or coated with a lubricious coating to facilitate the passage of instruments, tools, or other catheters through the internal lumen.

The everting balloon system 2 can have a media volume 8. The media volume 8 can be the contiguous open volume between the inner catheter 12 and outer catheter 6 that is proximal to the balloon membrane. A radially outer terminal perimeter of the balloon membrane 4 can be attached to the distal terminal end of the outer catheter 6. A radially inner terminal perimeter of the balloon membrane 4 can be attached to the distal terminal end of the inner catheter 12.

Figure 1A illustrates that the everting catheter system 104 can be in an unpressurized configuration. The media volume 8 can be uninflated and unpressurized. The balloon membrane 4 can be slack.

Figure 1B illustrates that that everting catheter system 104 can be in a pressurized and uneverted configuration. A pressurization device, such as a pump, for example at the proximal end of the everting catheter system 104 can be in fluid communication with the media volume 8. The pressurization device can deliver a fluid media, such as a pneumatic gas or hydraulic liquid media (e.g., saline, water, culture media, air, carbon dioxide, air-infused fluids, carbonated fluids, or combinations thereof), at a media pressure 14 to the media volume 8. The media pressure 14 in the everting balloon 22 can be from about 2 to about 5 atmospheres of pressure when in the everted configuration and higher media pressure 14 from about 5 atmospheres to 10 atmospheres are possible, for example, to provide greater everting capability for more difficult or stenotic passageways in the body.

The balloon membrane 4 can inflate and be in tension. The balloon membrane 4 can block the distal port of the inner catheter lumen 10.

Figure 1C illustrates that the everting catheter system 104 can be in an inflated and partially everted configuration. The inner catheter 12 can be translated distally, as shown by arrow, with respect to the outer catheter 6, and out of the outer catheter 6. The distal terminal end of the inner catheter 12 can be proximal of the distal terminal end of the balloon membrane. The distal terminal end of the inner catheter 12 can be proximal or terminal of the distal terminal end of the outer catheter 6. The balloon membrane 4 can block the distal port of the inner catheter lumen 10 or can be open allowing fluid communication between the inner catheter lumen 10 and the target site.

Figure 1D illustrates that the everting catheter system 104 can be in an inflated, fully everted, and fully distally extended configuration. The inner catheter 12 can be translated distally, as shown by arrow, with respect to the outer catheter 6 until the distal terminal end of the inner catheter 12 is longitudinally beyond or co-terminal with the distal terminal end of the balloon membrane. The distal port of the inner catheter 12 lumen can be unobstructedly accessible and in fluid communication with the target site.

In the fully inflated configuration, the balloon membrane 4 can form an inflated everting balloon. The everting balloon 22 can have a balloon outer diameter 20 and balloon length 18 in the inflated and fully everted configuration.

The balloon outer diameter 20 can be from about 2 mm to about 20 mm, more narrowly from about 2 mm to about 7 mm, for example about 3.0 mm. The outer diameter can be constant or vary along the length of the everting balloon. For example, for use in the cervical canal, the most proximal portion of the everting balloon 22 outer diameter could be configured with a smaller outer diameter than the remainder of the everting balloon membrane 54. As an example, the first proximal portion of the everting balloon 22 can have a smaller balloon outer diameter 20 such as from about 2 mm to 4mm for a length of from about 5 mm to about 10mm from the distal terminal end of the outer catheter 6, and the remainder of the length (e.g., from about 4 cm to about 7 cm along the everting balloon) of the everting balloon 22 can have a balloon outer diameter 20 from about 4 mm to about 7 mm.

The interior surface and lumen of the balloon can be coated with a lubricious material to facilitate rolling and unrolling of the interior surfaces of the everting balloon membrane 54.

The exterior surface of the balloon membrane 4 can be configured with ridges, projections, bumps, grooves, and additional surface or mechanical features, or combinations thereof, for example for increased friction or holding power within the vessel.

The everting balloon 22 length can be from about 2 cm to about 10 cm, more narrowly from about 3.5 cm to about 8.5 cm (e.g., for use in a longer uterine cavity lengths), yet more narrowly from about 5 cm to about 7.5 cm.

Figure 1E illustrates that the everting catheter system 104 can be in an inflated and partially or fully everted configuration. A tool 26, liquid, gas, or combinations thereof can be translated, as shown by the arrow, through the inner catheter lumen 10, out of the distal port of the inner catheter lumen 10 and into the target site. The tool 26 can be a biopsy tool, a scope, a sonogram probe, a plug, a cauterization tool, or combinations thereof. Suction can be applied from the proximal end of the inner catheter lumen 10, and to the target site, for example removing debris from the target site through the inner catheter lumen 10. For use in IVF procedures, an embryo transfer catheter 28 is translated through the inner catheter lumen 10 for deposition of embryo(s) or other reproductive material such as gametes or sperm.

To retract and reposition or remove the balloon membrane, the inner catheter 12 can be pulled proximally to pull the balloon membrane 4 back within the outer catheter 6. The balloon membrane 4 can be deflated or have media pressure 14 reduced and the entire system can be withdrawn from the target site.

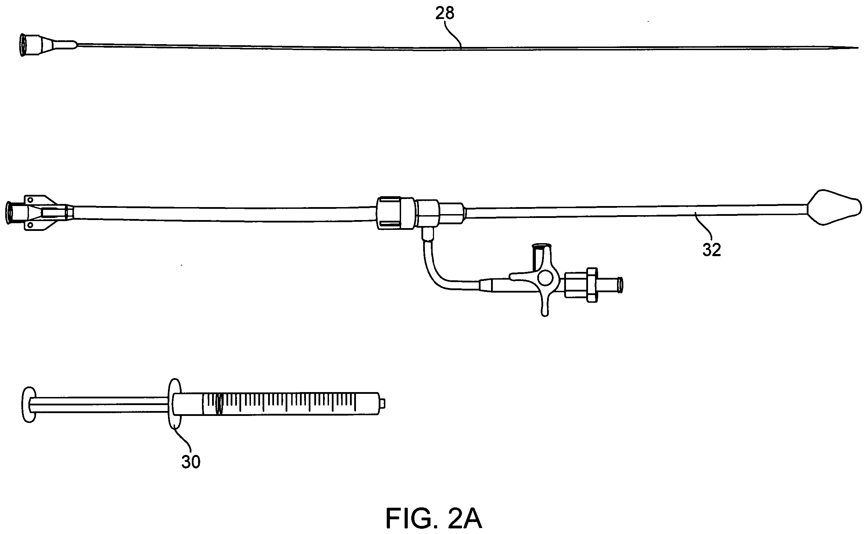

Figure 2A illustrates an everting balloon system 2 with a delivery catheter 32, embryo transfer catheter 28, and a pressurization syringe 30.

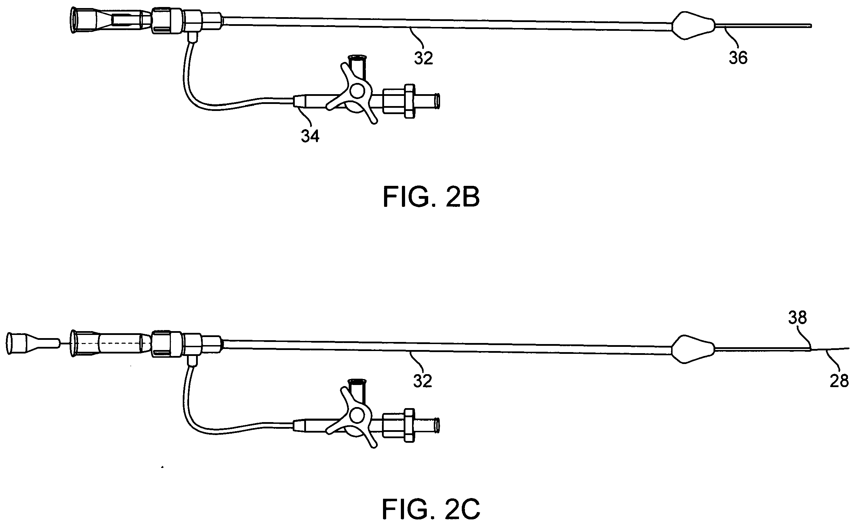

Figure 2B illustrates a variation of the everting balloon system 2 in a fully everted configuration. The everting balloon system 2 can be equipped with a distal end opening or a pre-determined valve.

Figure 2C illustrates an embryo transfer catheter 28 distally beyond the distal end of the everting balloon membrane 54. The everting catheter system 104 can access a bodily cavity (e.g., the uterine cavity or fallopian tubes) to deliver or introduce tools (e.g., instruments), reproductive media or material (e.g., embryos, in vitro fertilization (IVF) or insemination products, such as hormones), contrast media, dye, therapeutic agents, sclerosing agents to treat the endometrium, insufflation media, or combinations thereof to the cavity. For example, reproductive media can be delivered with a transfer catheter inserted through the inner catheter lumen 10 to the uterine cavity.

Figure 2B illustrates that a transfer catheter or insemination catheter can have a transfer connector 34, such as a female luer connector, a strain relief length, and a transfer tube. The transfer tube can hold the reproductive media. A delivery force, for example a positive fluid pressure, can be delivered through the transfer connector 34 and strain relief length to push the contents of the transfer tube into the target site.

The transfer catheter can attach to or inserted through the inlet port. The transfer tube can hold an embryo, for example for in vitro fertilization or IVF. The embryo transfer catheter 28 can deliver embryos through the system and to the uterine cavity and other agents that help facilitate embryo implantation such as materials that promote adherence of the embryo to the uterine endometrium. The embryo transfer catheter 28 can have a distal end configuration that can promote implantation of the embryo(s) within the endometrial wall or within the sub-endometrial surface.

The embryo transfer catheter 28 can hold spermatozoa and deliver the spermatozoa through the system and to the uterine cavity for intrauterine insemination procedures. The transfer catheter can hold and deliver or deposit materials, such as drugs, therapeutic agents, instruments, endoscopes, cytology brushes, other catheters, or combinations thereof through the system and into the uterine cavity. The transfer catheter can be connected to a vacuum source for the aspiration of materials from the uterine cavity or other bodily cavities and lumens.

The transfer catheter and/or materials can be loaded in the inner catheter lumen 10 prior to everting the everting balloon 22 within the vessel or bodily cavity. For example in the case of delivery of reproductive material in the uterine cavity, the transfer catheter can be loaded with washed and prepared semen in the transfer tube and the transfer catheter can be placed in the inner catheter lumen 10.

The inner catheter 12 can be extended and the everting balloon 22 can evert and unroll through the cervix and into the uterine cavity. Concurrently or subsequently, the transfer catheter can be advanced through the inner catheter lumen 10 into the uterine cavity. Once fully everted or when the transfer catheter becomes extended or exposed from the inner catheter 12 and beyond the everting balloon membrane 54, the reproductive material in the transfer catheter can be deposited by a syringe, squeeze bulb, piston, or other pressure system. A second delivery catheter 32, such as a second insemination, IVF, or drug delivery catheter 32 can be concurrently inserted into the inlet port or a second inlet port. The second delivery catheter 32 can be deployed to the target site concurrent with or subsequent to the transfer catheter. The embryo transfer catheter 28 can advance distally within the everting balloon 22 and the inner catheter lumen 10. The transfer catheter can deposit the reproductive material (e.g., sperm) within the uterine cavity.

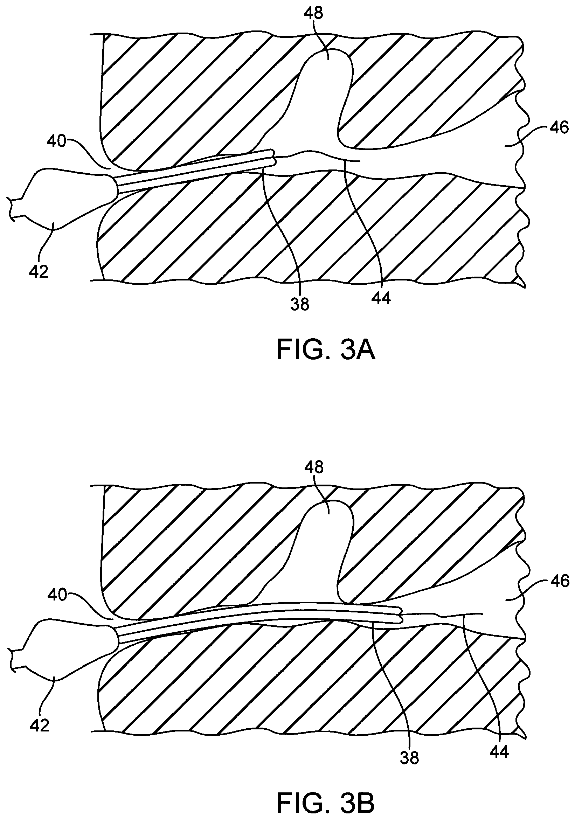

Figure 3A illustrates a cross-sectional view of a flexible tip guidance wire extending beyond the distal terminal end of the everting balloon membrane 54 during the eversion process directing the everting balloon system 2 beyond a cul-de-sac 48 in the endocervical canal or endocervix. (The everting balloon membrane distal end 38 can be at or immediately adjacent to the cul-de-sac 48 in the endocervical canal.) The flexible tip guidance wire distal end 44 can be translatably advanced beyond the opening of the cul-de-sac 48 and is positioned within the entrance or opening towards or within the uterine cavity. The delivery catheter 32 system can be equipped with a flexible tip guidance wire that allows the physician to steer or direct the leading edge of the balloon to the correct path within the uterus, for example, to facilitate access within the uterine cavity and through the cervical canal. The delivery catheter 32 system can be used, for example, when a defect, such as a C-section defect or scar, cul-de-sac 48, or crypt is present within the endocervix. Such defects can be visible via transabdominal or transvaginal ultrasound. The echogencitiy of the delivery catheter 32 is enhanced by pressurization fluid, or air, or a combination of both that creates echogenic density differences that are visualized by ultrasound. The flexible tip guidance wire can be introduced beyond the cul-de-sac 48 opening and towards the uterine cavity or target site, for example, to avoid the defect or cul-de-sac 48. The internal balloon pressure can be reduced or eliminated, for example, to advance the flexible tip guidance wire beyond the distal end of the everting balloon membrane 54. With everting balloon 22 pressure low or at zero, the flexible tip guidance wire can be threaded through the deflated balloon membrane 4 and advanced beyond the cul-de-sac 48 opening. Once the flexible tip guidance wire is advanced beyond the opening and towards the target site, the everting balloon membrane 54 pressure can be reestablished and the advancement of the inner catheter 12 can continue until the leading distal end of the everting balloon 22 moves past or distal to the cul-de-sac 48 opening.

Figure 3B illustrates a cross-sectional view of a flexible tip guidance wire distal end 44 distally beyond the everting balloon membrane distal end 38 at the completion of the eversion process beyond a cul-de-sac 48 in the endocervical canal. In this view, the everting balloon system 2 has been advanced towards and within the uterine cavity without entering the cul-de-sac 48. Once past the opening and towards the uterine cavity or target location, the flexible tip guidance wire can be removed once full eversion is complete, or prior to that by reflating the everting balloon 22 pressure to allow removal of the flexible tip guidance wire.

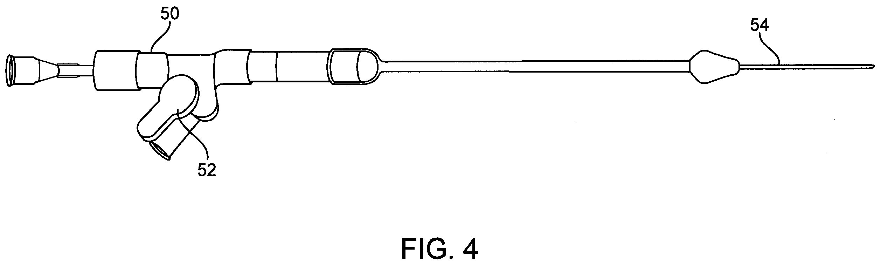

Figure 4 illustrates a variation of the everting balloon system 2 with a stopcock 52 configuration for maintaining pressurization. The everting balloon membrane 54 or system can be fully everted. The stopcock 52 is placed on the Y-fitting 50 connector 122 which can be used by the physician to hold the everting balloon system 2 during the procedure. The location of the stopcock 52 can provide finger-tip control of the pressurization state of the everting balloon system 2. At the completion of the eversion step for the inner catheter 12, the pressurization state of the everting balloon membrane 54 can be quickly removed. The removal of the pressurization state can occur prior to, during, or after the insertion of the embryo transfer catheter 28. Alternatively, the removal of the pressurization state can occur prior to, during, or after the deposition of the embryo(s) from the embryo transfer catheter 28. Yet further, the removal of the pressurization state can occur prior to, during, or after the removal of the embryo transfer catheter 28. Once the pressurization state is removed from the everting balloon system 2 and after the embryo(s) have been deposited within the uterine cavity, the entire everting balloon system 2 can be withdrawn from the uterine cavity.

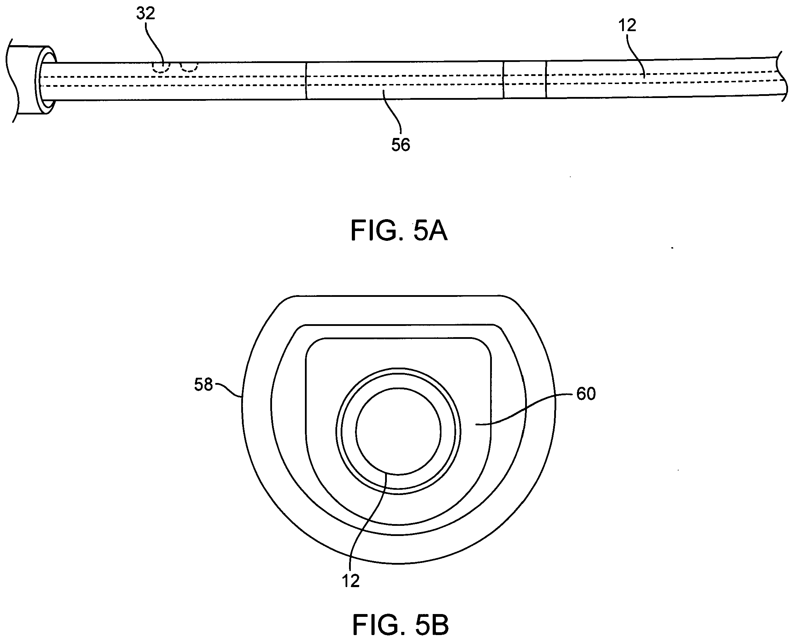

Figure 5A illustrates a variation of the everting balloon system 2 with an internal alignment mechanism 56 that can prevent rotation and spinning of the balloon membrane, for example, with respect to the delivery catheter 32. The internal alignment mechanism 56 can have or be an alignment piece 60. The internal alignment mechanism 56 can restrict or eliminate the twisting of the balloon system about itself and/or with respect to the delivery catheter 32. Multiple twists within the balloon system can hinder the advancement of the embryo transfer catheter 28 or other instruments and tools through the everting balloon system 2. The alignment piece 60 can be located within the outer tubing distal to the Y-fitting 50 and stasis valve that maintains pressurization within the everting balloon system 2 while the inner catheter 12 is being advanced or retracted during the eversion process.

Figure 5B illustrates a cross-sectional axial view of the internal alignment mechanism 56 and mating geometry of the delivery catheter 32 tubing. The radially inner and/or outer surface of the delivery catheter 32 outer tubing 58 can be D-shaped. The alignment piece 60 on the inner catheter 12 can be keyed (e.g., having a somewhat similar D-shape to the outer tubing) within the D-shape of the outer tubing to restrict or eliminate the rotation of the inner catheter 12 in relation to the outer tubing. The alignment piece 60 can be made from a material with a lubricous coating, Teflon, or other material that reduces the friction of the alignment piece 60 when being moved in the outer tubing.

The outer tubing outer and/or inner surface can have a D-shape, oval, elliptical shape, or combinations thereof, with a mating D-shape, oval, elliptical shape, or combinations thereof, on the alignment piece 60 that can restrict or eliminates the rotation of the inner catheter 12 in relation to the outer tubing.

The alignment piece 60 shape can be configured as the external surface throughout the entire inner catheter 12 tubing body. The shape of the external surface would in this configuration can mate with the internal geometry of the outer tubing. The surfaces can key into each other to restrict or eliminate the rotation of the inner catheter 12 to the outer tubing and the stasis valve can conform or fit to the external surface of the inner catheter 12 to maintain pressurization during the eversion process. As an example, the inner catheter 12 tubing can be configured with a rail surface or protrusion that mates or keys with one or more receptacles within the outer tubing internal geometry.

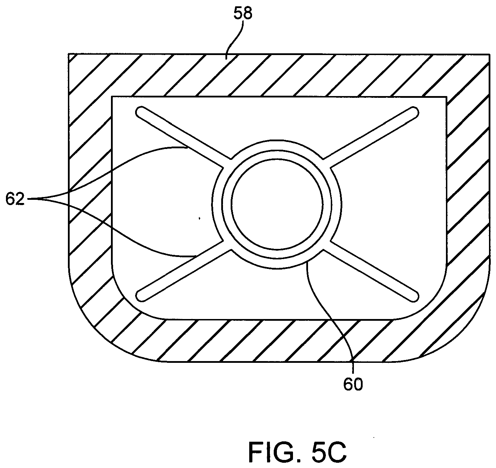

Figure 5C illustrates another embodiment of the alignment piece 60 configured as a spline that mates within the internal geometry of the outer tubing. The spline outer surfaces engage the internal geometry of the outer tubing to restrict or eliminate the rotation of the inner catheter 12 in relation to the outer tubing. The spline surfaces present minimal edges or corners that reduce the amount of surface area contacting the internal walls of the outer tubing. The reduction of surface area reduces the friction of the alignment piece 60 when moved within the outer tubing.

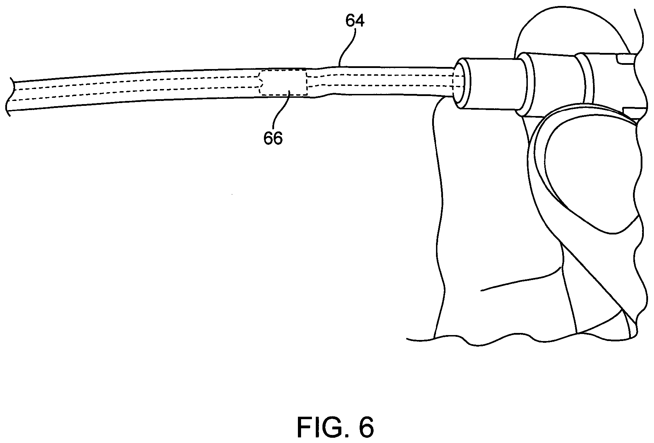

Figure 6 illustrates a variation of an everting balloon system 2 with an internal mechanism stopper 66 that prevents over-elongation of the inner catheter 12 during balloon inversion with a delivery catheter outer tubing crimp 64 on the outer tubing of the delivery catheter 32. In use during the eversion and inversion procedure, or during the preparation of the everting balloon system 2, the end user or physician can inadvertently retract the everting balloon system 2 and over-extend the balloon membrane. The over-extension can stretch, weaken, or damage the balloon membrane. Visual indicators or markings are useful but may not prevent over-extension if the end user is not diligent or is within a setting in which the indicia is readily visual. The stopper is located on the inner catheter 12 tubing body and is positioned at point where full inversion has occurred. At full inversion, the stopper can contact a mechanical detent, crimp, stop, or the distal end of the Y-fitting 50 connection, and prevents further retraction of the inner catheter 12 thereby eliminating the over-extension of the balloon membrane 4 beyond the full inversion state. The stopper could mechanically contact other mechanical structures built into the outer tubing such as a crimp as shown in Figure 6, or a reduction in internal diameter of the outer tubing, in which the stopper would engage the crimp or reduction in internal diameter of the outer tubing to physically prevent further retraction of the inner catheter 12 beyond the full inversion state.

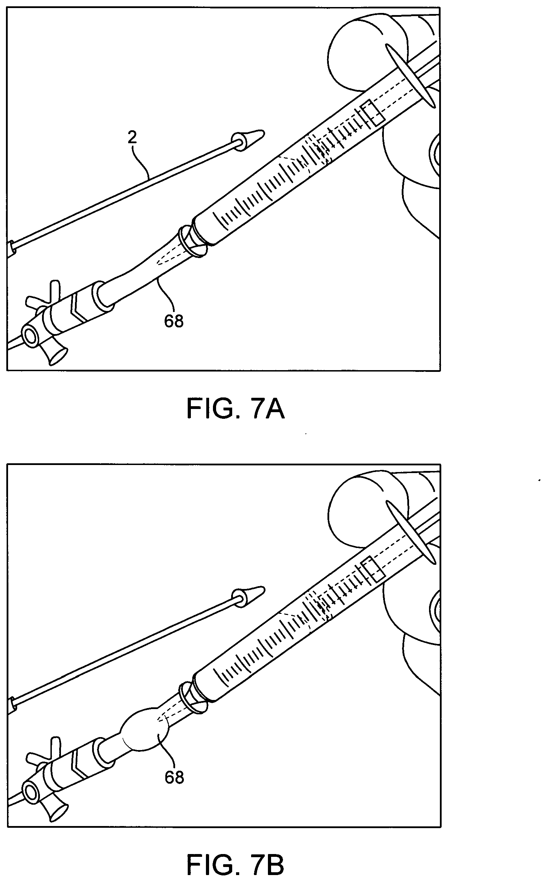

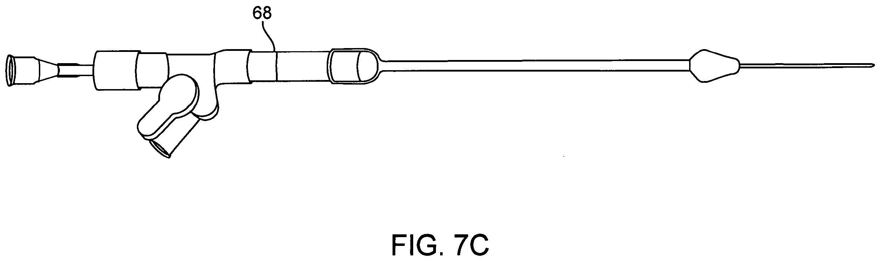

Figures 7A, 7B and 7C illustrate everting balloon system 2 with a compliant pressurization apparatus that can provide a pre-determined pressure within the catheter system with an indicator to the user that system is at the appropriate operating pressure.

Figure 7A illustrates a compliant member 68 built within the everting balloon system 2. The compliant member 68 can be filled or instilled with a fluid 76 from a syringe attached to the compliant member 68. The compliant member 68 can be configured as a separate component or accessory to assist the end user in preparing the everting balloon system 2. As the pressurization of the everting balloon system 2 occurs, compliant member 68 inflates and becomes a visual indicator that the system contains pressure. The compliant member 68 can be made from silicone tubing or balloon. Other elastomeric materials such as polyurethane, rubber, latex, configured as tubing or balloons are possible.

Figure 7B illustrates that the compliant member 68 can expand radially and lengthwise upon the influence of instilled fluid media under pressure. The expansion of the tubing walls of the compliant member 68 can dampen the fluid pressure within the everting balloon system 2. This can allow for variances in the amount of fluid 76 instilled by the end user that could impact the pressure rise in the everting balloon system 2. The amount of air in the everting balloon 22 can provide some compliance to the everting balloon system 2 and hence the compliant member 68 can provide a range of fluid volumes without exceeding the recommended working pressure of the everting catheter system.

The everting catheter system 104 can operates in a pressure range, for example, of about 2 to 4 atmospheres of pressure with a nominal pressure of about 3 atmospheres. For advancement within the cervical canal and into the uterine cavity, removing any residual air within the everting balloon system 2 can be performed before, during, and/or after the eversion process. This can be used, for example, in situations with tight or stenotic cervices. To achieve a working pressure of 3 atmospheres, a pressure gauge and/or inflation device (e.g., with a pressure gauge) can be connected to the everting balloon system 2. To achieve a working pressure of 3 atmospheres, an exact fluid volume amount can be prescribed to the everting balloon system 2 that can be instilled by the end user prior to end use. This can accomplish a working pressure of 3 atmospheres, for example, by measuring fluid volumes and the amount of air in the everting balloon system 2. The attachment of the compliant member 68 to the everting balloon system 2 can accomplish consistent fluid pressures within a wide range of fluid volumes, for example, providing a large tolerance to end user diligence during the catheter preparation process.

For example, a compliant member 68 can be attached to an everting balloon system 2 with a recommended fill volume of 3 cc of fluid 76. For test purposes while measuring preparing the everting catheter system 104 with varying amounts of fluid volume, the internal pressure of the system does not alter (much) beyond the nominal pressure of 3 atmospheres and in all fluid volumes, even when the fluid volume is intentionally doubled beyond the instructed amount, the internal pressure of the everting balloon system 2 remains within the operating working range of the system. For this test, the compliant member 68 can be constructed with 50 durometer silicone tubing with a .250" ID and a .500" OD and a 1.5 cm length of silicone tubing. At the ends of the silicone tubing can be male and female luer connectors 122 with attachment rings to mechanically adhere the silicone tubing to the luer connectors. In practice for this construction of the compliant member 68, as the silicone tubing is filled with fluid 76, the radial walls can expand and the overall length of the silicone tubing can increase in response to the increasing volume of fluid 76. Since the additional fluid volume can be accommodated by the compliant member 68, the internal fluid pressure of the everting balloon member can plateau at or near the desired nominal pressure amount.

| Fluid Volume Within Everting Balloon System and Compliant Member | Resultant Internal Pressure Within the Everting Balloon System |
|---|---|
| 3 cc of saline | 3.0 atmospheres |
| 4 cc of saline | 3.0 atmospheres |
| 5 cc of saline | 3.2 atmospheres |
| 6 cc of saline | 3.3 atmospheres |

As seen in the above data table, the resultant internal pressure can remain within the range of 2 to 4 atmospheres and at or near a nominal pressure of 3 atmospheres. In this set of experiments and with this configuration of compliant member 68, a fluid volume of 2X the amount yielded only a 10% increase in internal pressure. By altering the durometer, elastormeric properties, length and wall thickness of the compliant, other nominal pressure amounts can be obtained. The compliant member 68 can provide a safety margin against over-pressurization that can either damage the balloon system, and/or provide an everting balloon system 2 that operates outside of its operational working parameters. The compliant member 68 can be used in combination with a pressure relief valve in everting balloon system 2 that have more critical or tight pressure tolerances, or where internal pressure changes due to operator or anatomical factors can create internal pressures that go beyond the desired performance specification.

Figure 7C illustrates that the compliant member 68 can be located on the outer tubing of the everting balloon system 2. The compliant member 68 can be on the proximal end of the delivery catheter 32. The compliant member 68 can inflate with internal pressure. The location of the compliant member 68 can provide a visual indicator to the physician on the pressurization state of the everting balloon system 2 and impacts the internal pressure of the everting balloon system 2 due its compliance properties. The compliant member 68 can be made from silicone, polyurethane, PVC, rubber, latex, or other elastomeric material and can be shaped as a tube or a preformed balloon. In this location, the entire compliant member 68 can be held by the physician during use. In situations where a higher internal pressure is desired, the entire compliant member 68 can be grasped and squeezed while maintaining positional control of the everting balloon system 2. Squeezing the compliant member 68 circumferentially can create small rises in the internal pressure of the everting balloon system 2 that may be advantageous for advancing the everting balloon 22 through tight or narrow passages. In addition, relaxing the grasp of the compliant member 68 would instantly return the compliant member 68 and the everting balloon system 2 to the pervious operating pressure range. In some applications, the ability to pulse the compliant member 68 and thereby provide pulsatile pressure spikes within the everting balloon system 2 may be advantageous for tight or narrow passageways in which advancement of the everting balloon 22 is desired in small and discrete steps, or with minor increases in internal pressure.

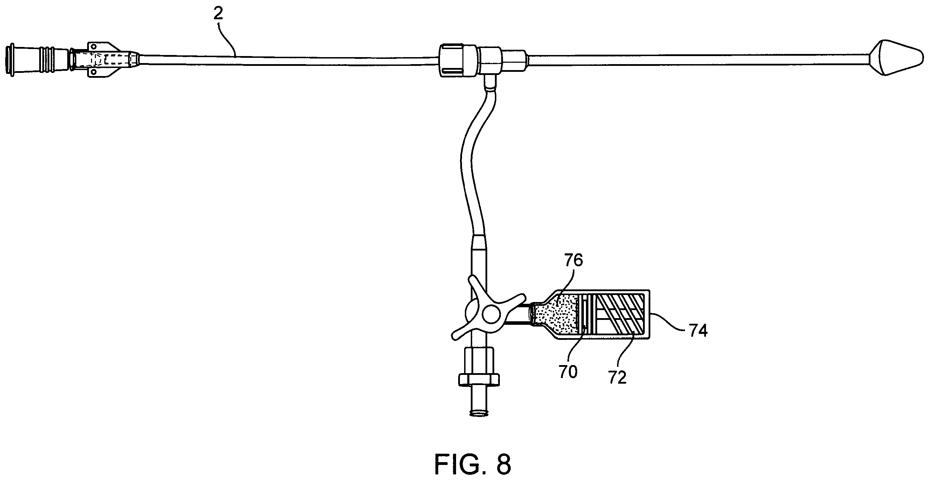

Figure 8 illustrates that the everting balloon system 2 can have a mechanism that can automatically pressurize the everting balloon system 2 to a predetermined amount. In a side view, a fluid cartridge 74 attached to and in controllable (e.g., via a luer lock or valve) fluid communication with the everting balloon system 2 can have a syringe plunger 70 and spring assembly 72. The fluid cartridge 74 can have a chamber that can be filled with a fluid 76 that can be used to supply internal pressure to the everting balloon. The syringe plunger 70 and spring assembly 72 can have a spring that can drive the plunger into the chamber with a known spring constant or K factor. The spring and k factor can be selected and configured to deliver a predetermined internal pressure to the everting balloon system 2. The spring can provide compliance to the everting balloon system 2 to maintain the internal pressure within the operating range and the spring, like the compliant member 68, can be responsive to changes in fluid volume, the everting balloon system 2 itself as it everts and inverts, and any anatomical forces acting on the everting balloon system 2.

The syringe plunger 70 and spring assembly 72 can be substituted for or used in combination with a syringe plunger 70 and air pressure canister in which the air canister with a predetermined internal gas pressure replaces the spring. Pressure from the air canister can act on the plunger and drive the fluid volume within the everting balloon system 2 to a predetermined internal pressure range. Air pressure canister can be prefilled with CO2 gas, air, other inert gas, or combinations thereof.

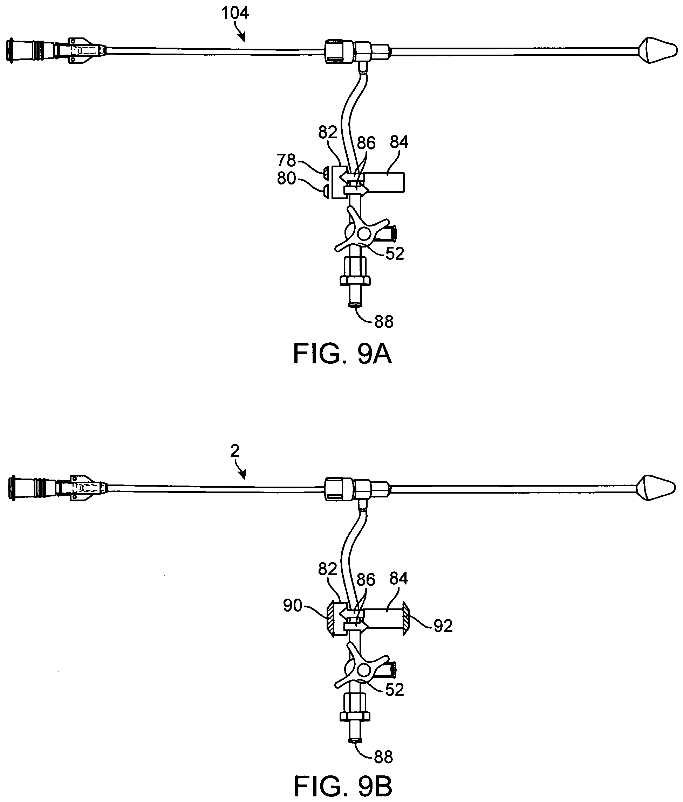

Figure 9A illustrates an everting balloon system 2 with an integral pressurization system that can provide an indicator and the ability to quickly shift the pressurization state of the balloon system from pressurized to non-pressurized at the fully everted state of the everting balloon system 2 via actuating trumpet valves for fluid 76 from constant pressure source 84 to a separate fluid reservoir 82. Actuation of trumpet valves can direct fluid 76 back into constant pressure source 84 through one-way valves 86. For example, a first trumpet valve 78 can release into the fluid reservoir 82, and a second trumpet valve 80 can return to a constant pressure source 84. A stopcock 52 is used to prepare and fill the everting balloon system 2 with fluid 76. The system can also have one-way valves 86. The one-way valves 86 can be within the trumpet valves (or the trumpet valves themselves) or can be separate from the trumpet valves. The everting balloon system 2 can have a fill port 88.

Figure 9B illustrates an everting balloon system 2 with an integral pressurization system that can provide an indicator and the ability to quickly shift the pressurization state of the balloon system from high pressurization to low pressurization, and back to high pressurization, or multiple intermediate states of pressurization, during the eversion process. A switch valve diverter and diaphragm 90 on the fluid reservoir 82 can open and close the fluid pathway into the everting balloon system 2 from the constant pressure source 84 and fluid reservoir 82. Depressing the plunger or diaphragm 90 of the fluid reservoir 82 returns the fluid 76 volume back into the everting balloon system 2 and in communication with the constant pressure source 84. While fluid 76 is diverted into the fluid reservoir 82, the internal pressure within the everting balloon system 2 drops to, or at, nears zero atmospheres. Depressing the diaphragm 90 plunger of the fluid reservoir 82 can push fluid 76 through a one-way valve 86 into the constant pressure source 84 chamber. Manual depression forces on the diaphragm 90 can be facilitated by the flexure of the diaphragm 90 surface from a convex profile to a concave profile as the fluid 76 is pushed through the one-way valve 86 and into the constant pressure force chamber. Fluid 76 going into the constant pressure force chamber can flow through a one-way valve 86 to enter the chamber. Once the diverter is flipped back to the everting balloon system 2, the constant pressure source 84 can instill the fluid 76 back into the everting balloon. Other combinations of one-way valves 86, check valves, or turn valves are possible to allow fluid pressure in the everting balloon system 2 to change from an operating pressure state or a zero pressure state quickly without having to reconnect to the everting catheter to a separately supplied fluid source, or without moving the position of the everting catheter within the bodily cavity.

The constant pressure source 84 could be configured to supply varying amounts of force for providing the internal pressure of the everting catheter system. The constant pressure source 84 can be supplied with a constant pressure regulator 92 that can modulate the amount of internal pressure being supplied to the everting balloon system 2. Pressure modulation can provide change from 3 atmospheres of pressure to 2, 1, or 0.5 atmospheres of pressure which can still provide the everting balloon 22 with structural shape but reduces the amount of eversion force, or the overall diameter of the everting balloon. For example, the everting balloon 22 can have its internal pressure modulated from 3 atmospheres of pressure at a point of nearly complete eversion but would then have the internal pressure modulated to 0.5 or 1 atmospheres of pressure as the embryo transfer catheter 28 is being loaded by the embryologist, or when the embryo transfer catheter 28 is being traversed through the inner catheter 12, or at as the entire everting catheter is inverted or removed from the uterine cavity without inverting the balloon back into the delivery catheter 32. Other degrees of pressure are possible with fingertip control of the physician without having to use an inflation device hooked up to the everting catheter system.

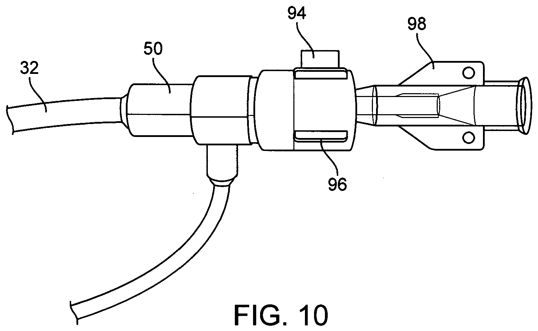

Figure 10 illustrates an everting balloon system 2 with a mechanism that stabilizes the inner catheter 12 at the full eversion stage and provides an indicator to the user that catheter system is at the appropriate step in the process for embryo transfer. The inner catheter 12 and the everting balloon 22 can reach full eversion when the inner catheter proximal hub 98 contacts the cap of the Y-fitting 50 of the delivery catheter 32. Receptacle on Y-fitting cap 96 is configured to accept and mate with the distal surface of the proximal hub. Contact of the inner catheter proximal hub 98 to the cap of the Y-fitting 50 elevates a pop up locking tab 94 as a visual indicator of the engaged position. (The pop up locking tab 94 is shown in the elevated and engaged position in Figure 10.) Mating action can be an audible or palpable, or both, as the two surfaces engage and lock. For the embryo transfer procedure, when the two surfaces engage and lock, the embryologist would provide the embryo transfer catheter 28 for traversing through the inner catheter 12. Depression of the pop up locking tab 94 to unlock would free the inner catheter proximal hub 98 from the mating surface. The two surfaces can also engage without locking, or engage with a mechanical or friction fit that can be overcome by slight retraction by the physician. As another embodiment, the mating action of the two surfaces could also mechanically open turn valve on the Y-fitting 50 to remove internal pressure within the everting balloon system 2 to reduce profile of the everting balloon 22 once the full eversion process is completed.

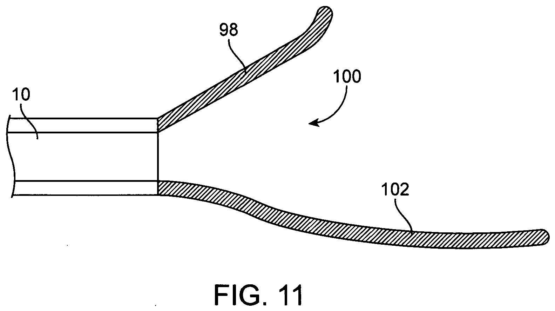

Figure 11 illustrates in a side cross-sectional view an everting balloon system 2 with a proximal hub connector that aids physician and embryologist in delivering the embryo transfer catheter 28 to the delivery catheter 32. The inner catheter proximal hub 98 can have a large funnel opening 100 to provide an easier target for the embryologist or the physician to place the distal end of the embryo transfer catheter 28 into the everting catheter system. Funnel of the proximal hub can also have a posterior extension 102 that provides a platform for resting the proximal end of the embryo transfer catheter 28 during the final steps of embryo transfer catheter 28 insertion, such as while inserting into the everting catheter. This may be particularly beneficial with embryology syringes that are heavy in weight, such as glass syringes, that could create extra downward forces on the embryo transfer catheter 28.

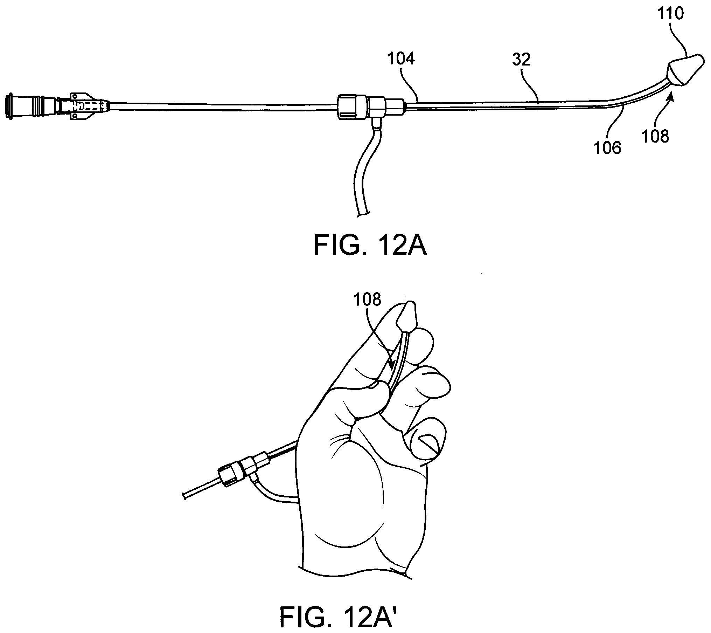

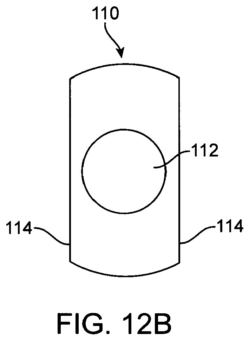

Figures 12A and 12A' illustrate an everting balloon system 2 shaped with distal end features that facilitate uterine access without the need for a speculum. During embryo transfer procedures, minimizing the manipulations to the patient's uterus, cervix, and vagina is both more comfortable to the patient but can also have a significant role in reducing the amount of uterine contractions that could spontaneously arise during a procedure as a result or response to the manipulations. Uterine contractions can have a deleterious effect to the implantation of embryos during a procedure. The insertion of a speculum itself has been demonstrated to elicit uterine contractions and is uncomfortable to the patient. The embodiment of the everting catheter system 104 in Figures 12A and 12B also facilitates the use of a transvaginal ultrasound probe during the embryo transfer procedure. Transvaginal ultrasound provides greater vision quality than an abdominal ultrasound in which the abdominal ultrasound probe needs to provide sound waves through the pelvic region of the patient which may have varying degrees of abdominal fat. Also abdominal ultrasound is enhanced by the patient having a full bladder which can also add to the discomfort to the procedure. The use of a transvaginal ultrasound probe during an embryo transfer procedure is difficult since existing embryo transfer catheter 28 systems require a speculum for insertion of the device into the cervix. The embodiment illustrated in Figures 12A and 12B is designed to provide rigidity to enter into the vagina and press off the posterior surface of the vagina. Angulation or curvature of the delivery catheter distal end 108 can direct the distal tip of everting catheter towards to cervical os. As illustrated in Figure 12B, acorn tip 110 is shaped for placement alongside the transvaginal ultrasound probe by having flat surfaces on either side of the acorn tip 110. In practice the physician would place the transvaginal probe into the vagina and alongside the cervix. The everting catheter system 104 would be introduced alongside the transvaginal probe until the acorn tip 110 is at the exocervix. The presence of the transvaginal ultrasound probe would create access, and in most cases, room in the vagina for visual confirmation of placement at the exocervix without the need for a speculum. The everting balloon 22 would be then placed into the endocervical canal. For an everting catheter system, the portion of the everting catheter that contacts the endocervix and uterine cavity is all contained within the delivery catheter 32 and does not contact the surfaces or fluids in the vagina, thus further obviating the need for a speculum during the procedure. Referring back to Figure 12A, posterior side of delivery catheter 32 has a curved flexure support 106. Flexure support 106 is designed to maintain distal end curvature for entry into the vagina and placement of the distal acorn tip 110 at the exocervix. Flexure support 106 has rigidity to push slightly downward in the vagina to retract vaginal tissues away from the cervix. Photo insert in Figure 12A shows the curvature of the delivery catheter 32.

Figure 12B illustrates a distal end with flat surfaces on both sides of the acorn tip 110 facilitate placement along either side of the transvaginal ultrasound probe regardless of the physician being right or left handed. Figure 12B illustrates the acorn tip 110 can have a distal end hole 112 and Flat sides 114.

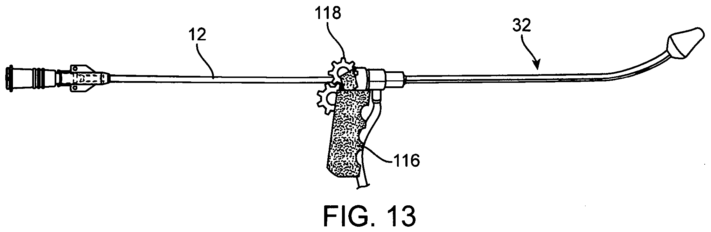

Figure 13 illustrates in a side view of an everting balloon system 2 with a handle 116 and controller mechanism 118 that controls the translation of the inner catheter 12. The handle 116 can minimize the amount of overall working length of the everting catheter system 104 without adding length to the everting catheter system. As an example, working space needed to place a handle 116 within an everting catheter system 104 can increase the overall length to the delivery catheter 32, inner catheter 12, and the embryo transfer catheter 28 that needs to be placed within the system. Adding length to these systems can create handling issues within the embryology laboratory, for example, in labs in which the loading of embryos within the embryo transfer catheter 28 is performed within a small incubator with side walls that will encroach on the handling of the embryology syringe and placement of the distal end of the embryo transfer catheter 28 within the embryo dish within the incubator. The handle 116 can reduce the amount of working length occupied by the handle 116 and controller mechanism 118 while still providing a one-handed operation to the advancement of the inner catheter 12 during use. The controller mechanism 118 can translatably advance and/or retract the inner catheter 12. The handle 116 can contain gear wheel controller mechanism 118 for engaging and translating the inner catheter 12 during use. The handle 116 has a posterior section that fits the palm and fingers of the physician without requiring the inner catheter 12 to be placed through the handle 116 portion for engagement with the controller mechanism 118. The handle 116 can be a pistol grip with gear wheels actuated by the thumb. The handle 116 can be incorporated into an everting catheter system 104 for use with transvaginal ultrasound.

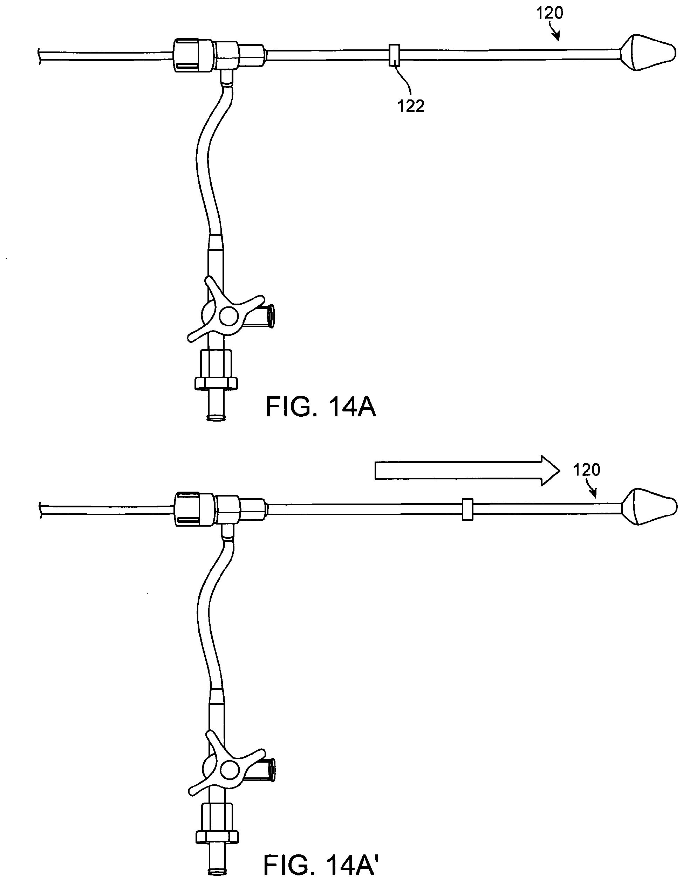

Figures 14A and 14A' illustrate an everting balloon system 2 with a translatable and adjustable distal end tip 120 that can alter the working length of the everting balloon. The translatable and adjustable distal end tip 120 can have a connector 122 on its proximal end and an acorn tip 110 on its distal end that can be advanced or retracted on the distal end of the delivery catheter 32. Advancement of the translatable and adjustable distal end tip 120, as shown in Figure 14A', can reduce the overall length of the everting balloon 22 within the bodily cavity without impacting the markings on the proximal end of the embryo transfer catheter 28.

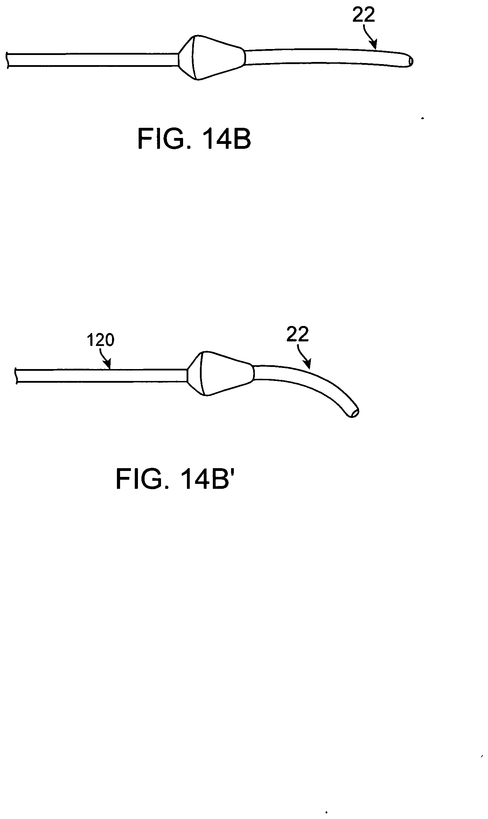

Figure 14B illustrates the translatable and adjustable distal end tip 120 at an extended position with resultant working length of the everting balloon 22 and everting balloon membrane 54. As an example, an everting balloon system 2 with a 5 cm long everting balloon 22 has the translatable and adjustable distal end tip 120 advanced 3 cm on the delivery catheter 32. As shown in Figures 14B', the resultant new working length of the everting balloon 22 in the body cavity is 2 cm. The connector 122 on the proximal end of the translatable and adjustable distal end tip 120 can be rotated to engage edges of D-shape tubing of the delivery catheter 32. Once rotated in the locked position, the translatable and adjustable distal end tip 120 can be configured to no longer move or slide on the delivery catheter 32. Unlocking the connector 122 by rotation can return movement to the translatable and adjustable distal end tip 120. Other types of connectors 122 can include twist valves that resist movement by friction on the outer tube of the delivery catheter 32 and can be untwisted to allow movement. Another example of a connector 122 is a clip that has an engaged and disengaged position which is actuated by the user.

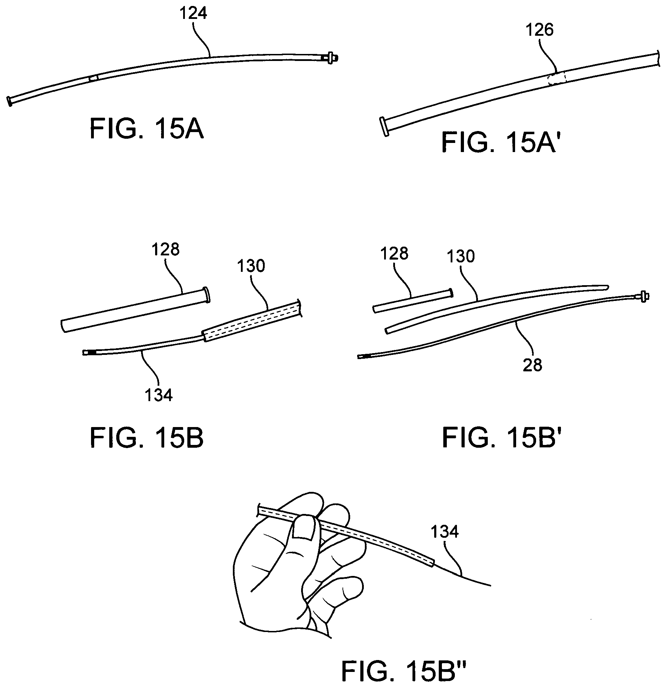

Figures 15A and 15A' illustrate a protective tube system 124 for the embryo transfer catheter 28 that can facilitate handling and transport of the catheter. The protective tube can be shipped to the user assembled with two tube components with male and female connections 126 attached to each other with the embryo transfer catheter 28 within the lumen of the protective tube.

Figures 15B, 15B' and 15B" illustrate a protective tube system 124 for the embryo transfer catheter 28 in the detached configuration for the loading of embryos. The female connection of the tube component at a female end 128 is separated from the male connection at a male end 130 at a point near the embryo transfer catheter distal end 134, leaving a distal length of the embryo transfer catheter 28 exposed for working under microscopic vision and the loading of embryo(s) and/or reproductive materials. Figure 15B" shows the handling of the embryo transfer catheter 28 when loading embryos with an embryology syringe.

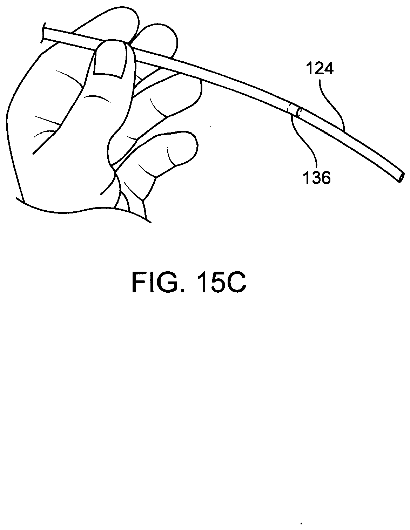

Figure 15C illustrates a protective tube system 124 for the embryo transfer catheter 28 in the re-attached mode for the transport of the embryo transfer catheter 28. Once loaded with embryo(s) and reproductive materials, the female connection can be reattached to the male connection for transport to the patient and the delivery catheter 32 system. The distal end of the embryo transfer catheter 28, for example with the reproductive material, can be radially covered by the protective tube system 124 when the female end 128 is attached to the male end 130. The reattachment connection point 136 in the protective tube between the male and female end 128 can be separated, for example, to provide a mechanism to gain access to the distal end of the embryo transfer catheter 28 for manipulation under a microscope or within an embryology incubator, and further be reattached for transport of the reproductive material to the patient and the completion of the embryo transfer procedure.

U.S. Patent Nos. 9,028,401, issued May 12, 2015; 9,101,391, issued August 11, 2015, 9,949,756, issued April 24, 2018; U.S. Patent Application Nos. 14/495,726, filed September 24, 2014; 14/525,043, filed October 27, 2014; and U.S. Provisional Application Nos. 61/902,742, filed November 11, 2013; 61/977,478, filed April 9, 2014; 62/005,355, filed May 30, 2014; and 62/007,339, filed June 3, 2014.

Any elements described herein as singular can be pluralized (i.e., anything described as "one" can be more than one). Any species element of a genus element can have the characteristics or elements of any other species element of that genus. The media delivered herein can be any of the fluids 76 (e.g., liquid, gas, or combinations thereof) described herein. Some elements may be absent from individual figures for reasons of illustrative clarity. The above-described configurations, elements or complete assemblies and their elements for carrying out the disclosure, and variations of aspects of the disclosure can be combined and modified with each other in any combination. All devices, apparatuses, systems described herein can be used for medical (e.g., diagnostic, therapeutic or rehabilitative) or non-medical purposes.

## Claims

1. A system for delivering matter into the reproductive tract of a female comprising:
a first catheter (6) having a lumen and a distal lumen port, wherein the first catheter (6) has a retracted configuration and an extended configuration;
an everting balloon (22) attached to the first catheter (6), wherein at least a length of the everting balloon (22) extends past the distal end of the first catheter (6) when the first catheter (6) is in the extended configuration; and
a second catheter (12) slidably located in the first catheter (6);
**characterized in that**
the second catheter (12) comprises an alignment piece (60) to prevent rotation of the second catheter (12) in relation to the first catheter (6), wherein the alignment piece (60) is keyed to mate with an internal geometry of the first catheter (6) to prevent rotation of the second catheter (12) in relation to the first catheter (6); and
a compliant member (68) is configured to maintain an internal pressure of the everting balloon (22) of 202,65 to 405,3 kPa (2 to 4 atmospheres) when the everting balloon (22) everts.

2. The system of claim 1, further comprising a stopping piece (66) to prevent over extension of the everting balloon (22) when the second catheter (12) is retracted in the first catheter (6) during inversion of the everting balloon (22).

3. The system in claim 1, wherein the compliant member (68) is constructed from a resilient tubing material within the operating range of the system.

4. The system in claim 3, wherein the compliant member (68) is made from silicone tubing.

5. The system in claim 1, wherein the compliant member (68) is placed onto the first catheter (6) and provides a visual indication of the internal pressure of the system.

6. The system in claim 5, wherein the compliant member (68) can provide additional pressure to the everting balloon (22) by manually depressing or squeezing the compliant member (68) during operation.

7. The system of claim 1, wherein the alignment piece (60) is D-shaped, and wherein the inner surface of the first catheter (6) is D-shaped.

8. The system of claim 1, further comprising a tool (26) advanceable through the second catheter (12), wherein the tool (26) comprises a therapeutic agent.

## Patentansprüche

1. System zur Abgabe von Material in den Fortpflanzungstrakt einer Frau, umfassend:
einen ersten Katheter (6) mit einem Lumen und einem distalen Lumenport, wobei der erste Katheter (6) eine zurückgezogene Konfiguration und eine ausgefahrene Konfiguration aufweist;
einen Eversionsballon (22), der an dem ersten Katheter (6) befestigt ist, wobei zumindest eine Länge des Eversionsballons (22) über das distale Ende des ersten Katheters (6) hinausragt, wenn sich der erste Katheter (6) in der ausgefahrenen Konfiguration befindet; und
einen zweiten Katheter (12), der gleitend in dem ersten Katheter (6) angeordnet ist;
**dadurch gekennzeichnet, dass**
der zweite Katheter (12) ein Ausrichtungsstück (60) umfasst, um eine Rotation des zweiten Katheters (12) relativ zum ersten Katheter (6) zu verhindern, wobei das Ausrichtungsstück (60) kodiert ist, um mit einer Innengeometrie des ersten Katheters (6) zusammenzupassen, um eine Rotation des zweiten Katheters (12) relativ zum ersten Katheter (6) zu verhindern; und
ein nachgiebiges Element (68) derart konfiguriert ist, dass es einen Innendruck des Eversionsballons (22) von 202,65 bis 405,3 kPa (2 bis 4 Atmosphären) aufrechterhält, wenn der Eversionsballon (22) evertiert.

2. System nach Anspruch 1, ferner umfassend ein Anschlagstück (66), um eine Überstreckung des Eversionsballons (22) zu verhindern, wenn der zweite Katheter (12) im ersten Katheter (6) während der Inversion des Eversionsballons (22) zurückgezogen wird.

3. System nach Anspruch 1, wobei das nachgiebige Element (68) aus einem elastischen Schlauchmaterial innerhalb des Betriebsbereichs des Systems konstruiert ist.

4. System nach Anspruch 3, wobei das nachgiebige Element (68) aus Silikonschlauch hergestellt ist.

5. System nach Anspruch 1, wobei das nachgiebige Element (68) auf dem ersten Katheter (6) angeordnet ist und eine visuelle Anzeige des Innendrucks des Systems bereitstellt.

6. System nach Anspruch 5, wobei das nachgiebige Element (68) dem Eversionsballon (22) zusätzlichen Druck bereitstellen kann, indem das nachgiebige Element (68) während des Betriebs manuell niedergedrückt oder zusammengedrückt wird.

7. System nach Anspruch 1, wobei das Ausrichtungsstück (60) D-förmig ist und wobei die Innenfläche des ersten Katheters (6) D-förmig ist.

8. System nach Anspruch 1, ferner umfassend ein Werkzeug (26), das durch den zweiten Katheter (12) vorschiebbar ist, wobei das Werkzeug (26) ein therapeutisches Mittel umfasst.

## Revendications

1. Système destiné à délivrer de la matière dans le tractus reproducteur d'un sujet femelle, comprenant :
un premier cathéter (6) ayant un lumen et un orifice distal du lumen, dans lequel le premier cathéter (6) présente une configuration rétractée et une configuration étendue ;
un ballon d'éversion (22) fixé au premier cathéter (6), dans lequel au moins une longueur du ballon d'éversion (22) s'étend au-delà de l'extrémité distale du premier cathéter (6) lorsque le premier cathéter (6) est dans la configuration étendue ; et
un second cathéter (12) disposé de manière coulissante dans le premier cathéter (6) ; **caractérisé en ce que**
le second cathéter (12) comprend une pièce d'alignement (60) destinée à empêcher la rotation du second cathéter (12) par rapport au premier cathéter (6), dans lequel la pièce d'alignement (60) présente un profil de détrompage destiné à s'accoupler avec une géométrie interne du premier cathéter (6) afin d'empêcher la rotation du second cathéter (12) par rapport au premier cathéter (6) ; et
un élément souple (68) est configuré pour maintenir une pression interne du ballon d'éversion (22) de 202,65 à 405,3 kPa (2 à 4 atmosphères) lorsque le ballon d'éversion (22) s'éverse.

2. Système selon la revendication 1, comprenant en outre une pièce d'arrêt (66) destinée à empêcher une extension excessive du ballon d'éversion (22) lorsque le second cathéter (12) est rétracté dans le premier cathéter (6) pendant l'inversion du ballon d'éversion (22).

3. Système selon la revendication 1, dans lequel l'élément souple (68) est construit à partir d'un matériau tubulaire résilient dans la plage de fonctionnement du système.

4. Système selon la revendication 3, dans lequel l'élément souple (68) est constitué d'une tubulure en silicone.

5. Système selon la revendication 1, dans lequel l'élément souple (68) est placé sur le premier cathéter (6) et fournit une indication visuelle de la pression interne du système.

6. Système selon la revendication 5, dans lequel l'élément souple (68) peut fournir une pression supplémentaire au ballon d'éversion (22) par enfoncement ou compression manuels de l'élément souple (68) pendant le fonctionnement.

7. Système selon la revendication 1, dans lequel la pièce d'alignement (60) est en forme de D, et dans lequel la surface interne du premier cathéter (6) est en forme de D.

8. Système selon la revendication 1, comprenant en outre un outil (26) pouvant être avancé à travers le second cathéter (12), dans lequel l'outil (26) comprend un agent thérapeutique.
